(19) Europäisches Patentamt
European Patent Office
Office européen des brevets

(11) EP 0 671 429 B1

(12) EUROPEAN PATENT SPECIFICATION

(45) Date of publication and mention of the grant of the patent: **06.05.1999 Bulletin 1999/18**

(51) Int Cl.[6]: **C08G 73/02**, C08G 81/00, C08G 69/44, C08G 63/68, C08F 283/00

(21) Application number: **95105140.8**

(22) Date of filing: **05.01.1984**

# (54) Dense star polymers and a process for producing dense star polymers

Sternpolymere mit hoher Dichte an Endgruppen und Verfahren zur Herstellung solcher Polymere

Polymères en étoile ayant des groupes à grande densité et procédé de préparation de ces polymères

(84) Designated Contracting States:
**AT BE CH DE FR GB IT LI NL SE**

(30) Priority: **07.01.1983 US 456226**

(43) Date of publication of application:
**13.09.1995 Bulletin 1995/37**

(62) Document number(s) of the earlier application(s) in accordance with Art. 76 EPC:
**94102257.6 / 0 608 908**
**84100080.5 / 0 115 771**

(73) Proprietor: **THE DOW CHEMICAL COMPANY**
**Midland, Michigan 48674 (US)**

(72) Inventors:
- **Tomalia, Donald A.**
  **Midland, Michigan 48640 (US)**
- **Dewald, James R.**
  **Bay City, Michigan 48706 (US)**

(74) Representative:
**Huber, Bernhard, Dipl.-Chem. et al**
**Patentanwälte**
**H. Weickmann, Dr. K. Fincke**
**F.A. Weickmann, B. Huber**
**Dr. H. Liska, Dr. J. Prechtel, Dr. B. Böhm,**
**Kopernikusstrasse 9**
**81679 München (DE)**

(56) References cited:
**DE-A- 3 222 328**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 671 429 B1

## Description

This invention relates to a process for preparing polyether dendrimer alkali metal salts, the polyether dendrimer alkali metal salts obtainable by the process of the invention as well as applications therefor.

Organic polymers are generally classified in a structural sense as either linear or branched. In the case of linear polymers, the repeating units (often called mers) are divalent and are connected one to another in a linear sequence. In the case of branched polymers, at least some of the mers possess a valency greater than 2 such that the mers are connected in a nonlinear sequence. The term "branching" usually implies that the individual molecular units of the branches are discrete from the polymer backbone, yet have the same chemical constitution as the polymer backbone. Thus, regularly repeating side groups which are inherent in the monomer structure and/or are of different chemical constitution than the polymer backbone are not considered as branches, e.g., dependent methyl groups of linear polypropylene. To produce a branched polymer, it is necessary to employ an initiator, a monomer, or both that possess at least three moieties that function in the polymerization reaction. Such monomer or initiators are often called polyfunctional. The simplest branched polymers are the chain branched polymers wherein a linear backbone bears one or more essentially linear pendant groups. This simple form of branching, often called comb branching, may be regular wherein the branches are uniformly and regularly distributed on the polymer backbone or irregular wherein the branches are distributed in nonuniform or random fashion on the polymer backbone. See T. A. Orofino, Polymer, 2, 295-314 (1961). An example of regular comb branching is a comb branched polystyrene as described by T. Altores et al. in J. Polymer Sci., Part A, Vol. 3, 4131-4151 (1965) and an example of irregular comb branching is illustrated by graft copolymers as described by Sorenson et al. in "Preparative Methods of Polymer Chemistry", 2nd Ed., Interscience Publishers, 213-214 (1968).

Another type of branching is exemplified by cross-linked or network polymers wherein the polymer chains are connected via tetravalent compounds, e.g., polystyrene molecules bridged or cross-linked with divinylbenzene. In this type of branching, many of the individual branches are not linear in that each branch may itself contain groups pendant from a linear chain. More importantly in network branching, each polymer macromolecule (backbone) is cross-linked at two or more sites to two other polymer macromolecules. Also the chemical constitution of the cross-linkages may, vary from that of the polymer macromolecules. In this so-called cross-linked or network branched polymer, the various branches or cross-linkages may be structurally similar (called regular cross-linked) or they may be structurally dissimilar (called irregularly cross-linked). An example of regular cross-linked polymers is a ladder-type poly(phenylsilsesquinone) as described by Sorenson et al., supra, at page 390. The foregoing and other types of branched polymers are described by H. G. Elias in Macromolecules, Vol. I, Plenum Press, New York (1977).

More recently, there have been developed polymers having so-called star structured branching wherein the individual branches radiate out from a nucleus and there are at least 3 branches per nucleus. Such star branched polymers are illustrated by the polyquaternary compositions described in USP Nos. 4,036,808 and 4,102,827. Star branched polymers prepared from olefins and unsaturated acids are described in USP 4,141,847. The star branched polymers offer several advantages over polymers having other types of branching. For example, it is found that the star branched polymers may exhibit higher concentrations of functional groups thus making them more active for their intended purpose. In addition, such star branched polymers are often less sensitive to degradation by shearing which is a very useful property in formulations such as paints, in enhanced oil recovery and other viscosity applications. Additionally, the star branched polymers have relatively low intrinsic viscosities even at high molecular weight.

While the star branched polymers offer many of the aforementioned advantages over polymers having more conventional branching, it is highly desirable to provide polymers which exhibit even greater concentrations of functional groups per unit volume of the polymer macromolecule as well as a more uniform distribution of such functional groups in the exterior regions of the macromolecule. In addition, it is often desirable to provide polymers having macromolecular configurations that are more spheroidal and compact than are the star branched polymers. Furthermore, dendrimers in the form of polyether dendrimer alkali metal salts are desirable for use in highly alkaline or highly acidic media.

The polyether dendrimer alkali metal salts of this invention are based on a polymer having a polyvalent core that is covalently bonded to at least two ordered dendritic (tree-like) branches which extend through at least two generations according to the general formula

$$\text{(Core)}\left[\text{(Repeat Unit)}_{\frac{N_r^G-1}{N_r-1}}\left(\text{Terminal Moiety}\right)_{\frac{N_r^G}{2}}\right]_{N_c}$$

wherein G is the number of generations and $N_c$ represents the valency of the core compound and the Repeat Unit has a valency of $N_r + 1$ wherein $N_r$ is the Repeating Unit multiplicity which is at least 2. The density of terminal groups per

unit volume in the polymer is at least 1.5 times that of a conventional star polymer having similar core and monomeric moieties and a comparable molecular weight and number of core branches, each of such branches of the conventional star polymer bearing only one terminal group, and (3) a molecular volume that is no more than 60 percent of the molecular volume of said conventional star polymer as determined by dimensional studies using scaled Corey-Pauling molecular models. For purposes of this invention, the term "dense" as it modifies "star polymer" means that it has a smaller molecular volume than a conventional star polymer having the same molecular weight. The conventional star polymer which is used as the base for comparison with the dense star polymer is one that has the same molecular, same core and monomeric components and same number of core branches as the dense star polymer. In addition while the number of terminal groups is greater for the dense star polymer molecule than in the conventional star polymer molecule, the chemical structure of the terminal groups is the same.

The terminal groups are converted into alkali metal salts.

In a somewhat more limited and preferred aspect, the salts of this invention are based on a polymer having a novel ordered star branched structure (herein called starburst structure). Hereinafter this polymer having a starburst structure is called a dendrimer. Thus, a "dendrimer" is a polymer having a polyvalent core that is covalently bonded to at least two ordered dendritic (tree-like) branches which extend through at least two generations. As an illustration, an ordered second generation dendritic branch is depicted by the following configuration:

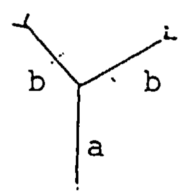


wherein "a" represents the first generation and "b" represents the second generation. An ordered, third generation dendritic branch is depicted by the following configuration:

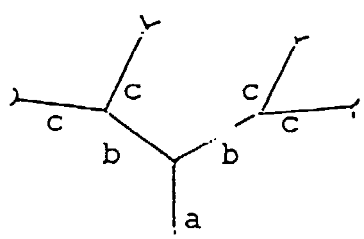


wherein "a" and "b" represent the first and second generation, respectively, and "c" represents the third generation. A primary characteristic of the ordered dendritic branch which distinguishes it from conventional branches of conventional polymers is the uniform or essentially symmetrical character of the branches as is shown in the foregoing illustrations. In addition, with each new generation, the number of terminal groups on the dendritic branch is an exact multiple of the number of terminal groups in the previous generation.

The present invention relates to a process for preparing a polyether dendrimer alkali metal salt by

(a) converting a polyvalent core $W(X)_n$ wherein W is a polyvalent core atom which is covalently bonded to nX reactive terminal OH-groups ($n \geq 2$) into its corresponding alkali metal salt by reaction of the polyvalent core with alkali metal hydroxide or zero valence alkali metal,

(b) reacting this salt with a molar excess of a partially protected multifunctional reagent T-(U) Ⓥ$_m$ wherein U represents a multivalent moiety covalently bonded to mⓋprotected moieties ($m \geq 2$) and to one T, a moiety capable of reacting with X to form W[(X'-T')UⓋ$_m$]$_n$, wherein X' and T' represent the residue of reaction between X and T,

(c) activating the obtained first generation compound by deprotecting it,

(d) converting the activated compound into alkali metal salt form by reaction with an alkali metal hydroxide or zero valence alkali metal, and, if desired,

(e) reacting the alkali metal salt with further partially protected reagent T-U-Ⓥ$_m$ to form the second generation protected dendrimer

(f) and repeating steps (c) and (d) to provide higher generation dendrimer alkali metal salts.

Other aspects of this invention are methods for using the dense star polymer alkali metal salts in such applications as demulsifiers for oil/water emulsions, wet strength agents in the manufacture of paper, agents for modifying viscosity in aqueous formulations such as paints and the like. For example, in a demulsification method, an emulsion of oil and water is contacted with a demulsifying amount of the dense star polymer under conditions sufficient to cause phase separation.

The dense star polymer-based salts of the present invention exhibit the following properties which are unique or are superior to similar properties of conventional star branched polymers and other branched polymers having similar molecular weight and terminal groups:

(a) greater branch density;
(b) greater terminal group density;
(c) greater accessibility of terminal groups to chemically reactive species; and
(d) lower viscosity.

In the dense star polymer-based salts of the present invention, the core is covalently bonded to at least two, most preferably at least three, core branches with each core branch having a calculated length of at least 3 Angstrom units (A) (0.3 nm), preferably at least 4 A (0.4 nm), most preferably at least 6 A (0.6 nm). These polymers preferably have an average of at least 2, more preferably at least 3 and most preferably at least 4 terminal groups per polymer molecule. Preferably, the core branches have a dendritic character, most preferably an ordered dendritic character as defined hereinafter. In preferred dense star polymers, the terminal groups are functional groups that are sufficiently reactive to undergo addition or substitution reactions. An Example of such functional groups is hydroxy. The dense star polymers differ from conventional star or star-branched polymers in that the dense star polymers have a greater concentration of terminal groups per unit of molecular volume than do conventional star polymers having an equivalent number of core branches and an equivalent core branch length. Thus, the density of terminal groups per unit volume in the dense star polymer is at least 1.5 times the density of terminal groups in the conventional star polymer, preferably at least 5 times, more preferably at least 10 times, most preferably from 15 to 50 times. The ratio of terminal groups per core branch in the dense polymer is at least 2, more preferably at least 3, most preferably from 4 to 1024. Preferably, for a given polymer molecular weight, the molecular volume of the dense star polymer is no more than 50 volume percent, more preferably from 16 to 50, most preferably from 7 to 40 volume percent of the molecular volume of the conventional star polymer.

The dendrimer-based salts of the present invention are characterized as having a polyvalent core that is covalently bonded to at least two ordered dendritic branches which extend through at least two generations. Such ordered branching can be illustrated by the following sequence wherein G indicates the number of generations:

G=1

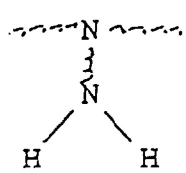

G=2

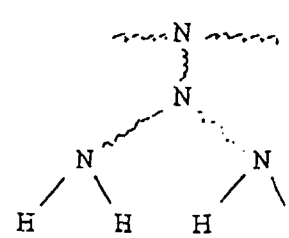

G=3

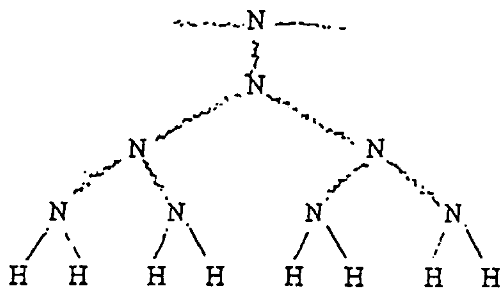

Mathematically, the relationship between the number of terminal groups on a dendritic branch and the number of generations of the branch can be represented as follows:

$$\text{\# of terminal groups per dendritic branch} = \frac{N_r^G}{2}$$

wherein G is the number of generations and $N_r$ is the repeating unit multiplicity which is at least 2 as in the case of amines. The total number of terminal groups in the dendrimer is determined by the following:

$$\text{\# of terminal groups per dendrimer} = \frac{N_c N_r^G}{2}$$

wherein G and $N_r$ are as defined before and $N_c$ represents the valency (often called core funtionality) of the core compound. Accordingly, the dendrimer-based salts of the present invention can be represented in its component parts as follows:

$$(\text{Core})\left[(\text{Repeat Unit})_{\frac{N_r^G-1}{N_r-1}}\begin{pmatrix}\text{Terminal}\\ \text{Moiety}\end{pmatrix}_{\frac{N_r^G}{2}}\right]_{N_c}$$

wherein the Core, Terminal Moiety, G and $N_c$ are as defined before and the Repeat Unit has a valency or functionality of $N_r + 1$ wherein $N_r$ is as defined before.

An illustration of a functionally active dendrimer of a ternary or trivalent core which has three ordered, second generation dendritic branches is depicted by the following configuration:

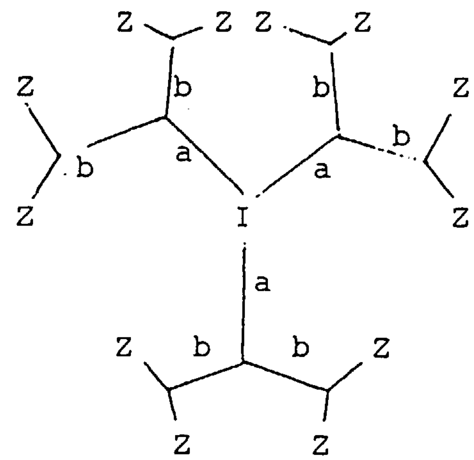

wherein I is a trivalent core atom or molecule having a covalent bond with each of the three dendritic branches, Z is a terminal moiety and "a" and "b" are as defined hereinbefore. An example of such a ternary dendrimer is polyamidoamine which is not claimed in this invention and which is represented by the following structural formula:

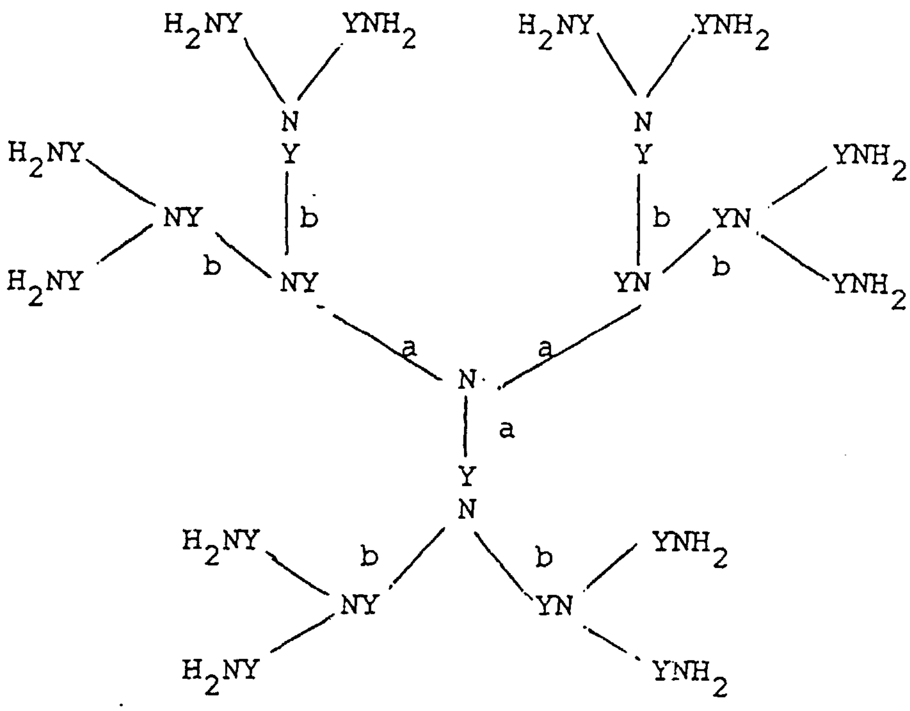

wherein Y represents a divalent amide moiety such as

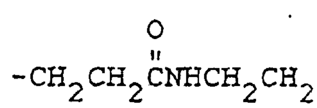

and "a" and "b" indicate first and second generations, respectively. In these two illustrations, $N_c$ is 3 and $N_r$ is 2. In the latter of the two illustrations, the Repeat Unit is YN. While the foregoing configuration and formula illustrate a trivalent core, the core atom or molecule may be any monovalent or monofunctional moiety or any polyvalent or polyfunctional moiety, preferably a polyvalent or polyfunctional moiety having from 2 to 2300 valence bonds or functional sites available for bonding with the dendritic branches, most preferably from 2 to 200 valence bonds or functional sites.

Accordingly, this dense star must have at least 2 generations in order to exhibit the desired density of terminal groups. Also, Y may be any other divalent organic moiety such as, for example, alkylene, alkylene oxide, alkyleneamine, with the depicted amide moiety being the most preferred. In addition to amine, the terminal groups of the dendrimer may be any functionally active moiety that can be used to propagate the dendritic branch to the next generation. Examples of such other moieties include carboxy, aziridinyl, oxazolinyl, haloalkyl, oxiranyl, hydroxy and isocyanato, with amine or carboxylic ester moieties being preferred. While the dendrimers preferably have dendritic branches having 2 to 6 generations, dendrimers having dendritic branches up to 12 generations are suitably made and employed in the practice of this invention.

The amidoamine dendimers are represented by the formula:

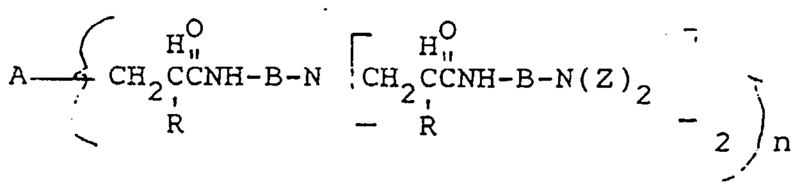

wherein A is a n-valent core derived from a nucleophilic compound, R is hydrogen or lower alkyl, B is a divalent moiety capable of linking amine groups, n is an integer of 3 or more corresponding to the number of the core branches and Z is hydrogen or

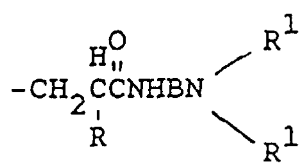

wherein $R^1$ is hydrogen or

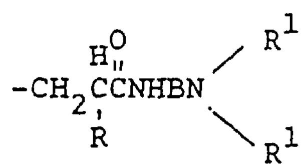

wherein each generation is represented by

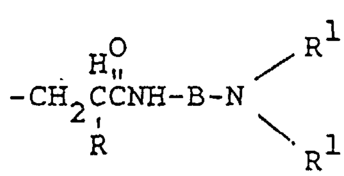

More preferably A is a core such as

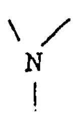 ,

$>NCH_2CH_2N<$ or

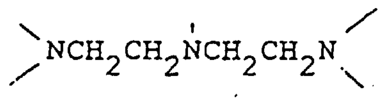 ;

R is hydrogen or methyl; B is the divalent residue of a polyamine, most preferably an alkylene polyamine such as ethylene diamine or a polyalkylene polyamine such as triethylene tetramine; n is an integer from 3 to 2000, more preferably from 3 to 1000, most preferably from 3 to 125, and Z is most preferably

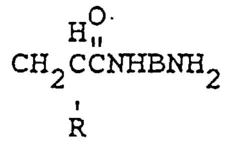 ,

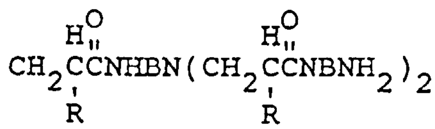

or

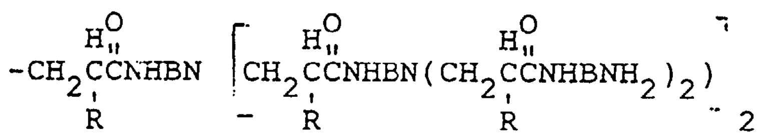 .

The dense star polymer-based salts of this invention are readily prepared by reacting a compound capable of generating a polyvalent core with a compound or compounds which causes propagation of dendritic branches from the core.

The compound capable of generating a polyvalent core, $W(X)_n$, wherein w is the polyvalent core atom and is covalently bonded to nX reactive terminal OH-groups ($n \geq 2$), is converted into alkali metal salt form by reaction of the polyvalent core with alkali metal hydroxide or zero valence alkali metal. The resulting salt is then reacted with a molar excess of a partially protected multifunctional reagent, T(U)Ⓥ$_m$, wherein U represents a multivalent moiety covalently bonded to mⓋ protected moieties ($m \geq 2$), and to one T, a moiety capable of reacting with X to form W[(X'-T')UⓋ$_m$]$_n$, wherein X' and T' represent the residue of reaction between X and T. This first generation compound is then subjected to activation conditions whereby the Ⓥ moieties are made reactive (deprotected) and converted into alkali metal salt form by reacting the activated compound with an alkali metal hydroxide or zero valence alkali metal. This alkali metal salt is further reacted with the partially protected multifunctional reagent, T-U-Ⓥ$_m$, to form the second generation protected dendrimer,

W[{(X'-T')UV}$_m$T'-UⓋ$_m$]$_n$.

This protected dendrimer can be activated and reacted again in a similar manner to provide the third generation protected dendrimer. Finally, the last generation protected dendrimer is converted into the alkali metal salt as described.

Illustrative of the partially protected reactant method of the present invention, a polyol such as pentaerythritol, $C(CH_2OH)_4$, is employed as the polyvalent core generating compound and is converted to alkali metal salt form, e.g., sodium or lithium, by reaction with alkali metal hydroxide or zero valence alkali metal and then reacted with a molar excess of a partially protected compound such as tosylate ester of 1-ethyl-4-hydroxymethyl-2,6,7-trioxabicyclo[2,2,2] octane to form a protected first generation polyether,

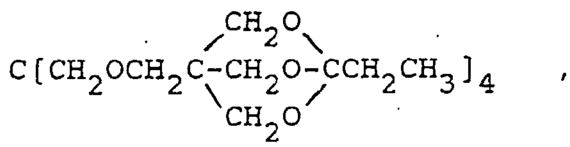

which is then activated by reacting with acid such as hydrochloric acid to form the unprotected first generation polyether, $C(CH_2O-CH_2C[CH_2OH]_3)_4$. This polyether is converted to alkali metal salt form by reaction with alkali metal hydroxide or zero valence alkali metal and then reacted with a molar excess of the partially protected tosylate ether to form the protected second generation polyether. The foregoing sequence is repeated as desired for additional generation development according to conditions and procedur s described in Endo et al., J. Polym. Sci., Polym. Lett. Ed., 18, 457 (1980), Yokoyama et al., Macromolecules, 15, 11-17 (1982), and Pedias et al. Macromolecules, 15, 217-223 (1982). Finally, the last generation protected dendrimer is converted into the alkali metal salt. These polyether dendrimer salts are particularly desirable for use in highly alkaline or highly acidic media wherein hydrolysis of a polyamidoamine dendrimer would be unacceptable.

Suitable nucleophilic cores include polyols such as the aforementioned pentaerythritol, ethylene glycol and polyalkylene polyols such as polyethylene glycol and polypropylene glycol; 1,2-dimercaptoethane and polyalkylene polymercaptans; thiophenols, and phenols. The polyols are preferred for the preparation of polyether dendrimers by the partially protected reactant method.

Thus prepared, the dendrimer salts can be reacted with a wide variety of compounds to produce the polyfunctional compounds having the unique characteristics that are attributable to the structure of the dendrimer.

In all instances, such derivatives of the dendrimer salts are prepared using procedures and conditions conventional for carrying out reactions of organic compounds bearing the particular functional group with the particular organic reactant.

## Claims

1. A process for preparing a polyether-dendrimer-alkali metal salt by

    (a) converting a polyvalent core $W(X)_n$ wherein W is a polyvalent core atom which is covalently bonded to nX reactive terminal OH-groups ($n \geq 2$) into its corresponding alkali metal salt by reaction of the polyvalent core with alkali metal hydroxide or zero valence alkali metal,

    (b) reacting this salt with a molar excess of a partially protected multifunctional reagent T-(U)Ⓥ$_m$ wherein U represents a multivalent moiety covalently bonded to mⓋ protected moieties ($m \geqq 2$) and to one T, a moiety capable of reacting with X to form W[(X'-T')UⓋ$_m$]$_n$, wherein X' and T' represent the residue of reaction between X and T,

    (c) activating the obtained first generation compound by de-protecting it,

    (d) converting the activated compound into alkali metal salt form by reaction with an alkali metal hydroxide or zero valence alkali metal, and, if desired,

    (e) reacting the alkali metal salt with further partially protected reagent T-U-Ⓥ$_m$ to form the second generation protected dendrimer

    (f) and repeating steps (c) and (d) to provide higher generation dendrimer alkali metal salts.

2. Process according to claim 1 wherein the polyvalent core $W(X)_n$ is a polyol.

3. Process according to claim 2 wherein the polyol is pentaerythritol.

4. Process according to claims 1, 2 or 3 wherein the partially protected multifunctional reagent T-(U)- V $_m$ is the tosylate ester of 1-ethyl-4-hydroxymethyl,2,6,7-trioxabicyclo [2,2,2] octane.

5. Process according to anyone of the foregoing claims wherein the activation is conducted by reacting with acid.

6. Polyether dendrimer alkali metal salt obtainable according to the method of any of claims 1 to 5.

7. Use of a polyether dendrimer alkali metal salt according to claim 6 in highly alkaline or highly acidic media.

**Patentansprüche**

1. Verfahren zum Herstellen eines Polyetherdendrimer-Alkalimetallsalzes durch:

   (a) Überführen eines polyvalenten Kerns $W(X)_n$, worin W ein polyvalentes Kernatom ist, das kovalent an nX reaktive endständige OH-Gruppen (n≥2) gebunden ist, in sein entsprechendes Alkalimetallsalz durch Reaktion des polyvalenten Kerns mit Alkalimetallhydroxid oder nullwertigem Alkalimetall,

   (b) Umsetzen dieses Salzes mit einem molaren Überschuß eines zum Teil geschützten multifunktionellen Reagens T-(U) $Ⓥ_m$, worin U eine mehrwertige Gruppe bedeutet, die kovalent an mⓋ geschützte Gruppen (m≥2) und an ein T gebunden ist, wobei eine Gruppe mit X reagieren kann, um $W[(X'-T')UⓋ_m]_n$ zu bilden, worin X' und T' den Rest einer Reaktion zwischen X und T bedeuten,

   (c) Aktivieren der erhaltenen Verbindung der ersten Generation durch Schutzgruppenentfernung,

   (d) Überführen der aktivierten Verbindung in die Alkalimetallsalzform durch Reaktion mit einem Alkalimetallhydroxid oder nullwertigem Alkalimetall und gegebenenfalls

   (e) Umsetzen des Alkalimetallsalzes mit weiterem zum Teil geschütztem Reagens T-U-$Ⓥ_m$, um das geschützte Dendrimer der zweiten Generation zu bilden,

   (f) und Wiederholen der Schritte (c) und (d), um Dendrimeralkalimetallsalze höherer Generation bereitzustellen.

2. Verfahren nach Anspruch 1, worin der polyvalente Kern $W(X)_n$ ein Polyol ist.

3. Verfahren nach Anspruch 2, worin das Polyol Pentaerythritol ist.

4. Verfahren nach den Ansprüchen 1, 2 oder 3, worin das zum Teil geschützte multifunktionelle Reagens $T-(U)-V_m$ der Tosylatester von 1-Ethyl-4-hydroxymethyl-2,6,7-trioxabicyclo[2,2,2]oktan ist.

5. Verfahren nach einem der vorhergehenden Ansprüche, worin die Aktivierung durch Reaktion mit einer Säure durchgeführt wird.

6. Polyetherdendrimer-Alkalimetallsalz, erhältlich mit dem Verfahren nach einem der Ansprüche 1 bis 5.

7. Verwendung eines Polyetherdendrimer-Alkalimetallsalzes nach Anspruch 6 in stark alkalischen oder stark sauren Medien.

**Revendications**

1. Procédé de préparation d'un sel de métal alcalin d'un polyéther dendrimère, lequel procédé consiste à

   a) convertir un coeur polyvalent $W(X)_n$, dans lequel W est un atome central polyvalent qui est lié par liaisons covalentes à n groupes hydroxy terminaux réactifs X (n ≥ 2), en le sel de métal alcalin correspondant, en faisant réagir le coeur polyvalent avec un hydroxyde de métal alcalin ou avec un métal alcalin à l'état zérovalent ;
   b) faire réagir ce sel avec un réactif polyfonctionnel partiellement protégé $T-(U)(V)_m$ en excès molaire, dans lequel U représente un fragment polyvalent lié par liaisons covalentes à m fragments protégés (V) (m ≥ 2) et à un seul fragment T capable de réagir avec X, pour obtenir $W[(X'-T')U(V)_m]_n$ où X' et T' représentent les résidus de la réaction entre X et T ;
   c) activer le composé de première génération ainsi obtenu, en le déprotégeant ;

d) convertir le composé activé en un sel de métal alcalin, en le faisant réagir avec un hydroxyde de métal alcalin ou avec un métal alcalin à l'état zérovalent ;

et si on le souhaite,

e) faire réagir ce sel de métal alcalin avec un supplément de réactif partiellement protégé T-(U)$(V)_m$, pour obtenir le dendrimère protégé de deuxième génération ;
f) et répéter les étapes (c) et (d) pour obtenir les sels de métal alcalin des dendrimères de générations d'ordre plus élevé.

**2.** Procédé conforme à la revendication 1, dans lequel le coeur polyvalent $W(X)_n$ est un polyol.

**3.** Procédé conforme à la revendication 2, dans lequel le polyol est du pentaérythritol.

**4.** Procédé conforme à la revendication 1, 2 ou 3, dans lequel le réactif polyfonctionnel partiellement protégé T-(U)$(V)_m$ est l'ester tosylate du 1-éthyl-4-hydroxyméthyl-2,6,7-trioxabicyclo[2,2,2]octane.

**5.** Procédé conforme à l'une des revendications précédentes, dans lequel on réalise l'activation par réaction avec un acide.

**6.** Sel de métal alcalin d'un polyéther dendrimère, qu'on peut obtenir selon un procédé conforme à l'une des revendications 1 à 5.

**7.** Utilisation d'un sel de métal alcalin d'un polyéther dendrimère, conforme à la revendication 6, dans des milieux fortement acides ou fortement alcalins.